Europäisches Patentamt

European Patent Office

Office européen des brevets

(19)

(11) Numéro de publication: **0 226 068
B1**

(12)

# FASCICULE DE BREVET EUROPEEN

(45) Date de publication du fascicule du brevet:
**28.12.88**

(51) Int. Cl.⁴: **A 61 F 5/01**

(21) Numéro de dépôt: **86116109.9**

(22) Date de dépôt: **21.11.83**

(60) Numéro de publication de la demande initiale en application
de l'article 76 CBE: **0143132**

(54) **Appareillage externe pour handicapé moteur de la main.**

(43) Date de publication de la demande:
**24.06.87 Bulletin 87/26**

(45) Mention de la délivrance du brevet:
**28.12.88 Bulletin 88/52**

(84) Etats contractants désignés:
**AT BE CH DE GB IT LI LU NL SE**

(56) Documents cités:
**DE-A- 2 615 209
DE-C- 299 882
FR-A- 2 340 080
FR-E- 20 530
GB-A- 1 150 072**

(73) Titulaire: **Salort, Guy, 219, rue Raymond Losserand,
F-75014 Paris (FR)**

(72) Inventeur: **Salort, Guy, 219, rue Raymond Losserand,
F-75014 Paris (FR)**

(74) Mandataire: **Peuscet, Jacques et al, Cabinet
Peuscet 68, rue d'Hauteville, F-75010 Paris (FR)**

ACTORUM AG

## Description

La présente invention a trait à un appareillage externe pour handicapé moteur de la main.

Le but de la présente invention est de fournir une orthèse autonome, qui réalise une mise en compression des articulations par des moyens neutralisant d'une façon dynamique le poids du membre. On permet, ainsi, l'information et la réactivation des unités motrices par une modulation de la chute, dans le temps et dans l'espace, du membre handicapé; on améliore et on accélère ainsi la récupération globale du geste volontaire de la préhension.

Jusqu'à ce jour, dans l'état de la technique, on a proposé des orthèses de membre supérieur de trois types: dans le premier type, les différentes parties du membre sont enserrées dans des coques que l'on manœuvre les unes par rapport aux autres au moyen d'organes moteurs commandés par le sujet, tels que, par exemple, un moteur électrique ou un muscle artificiel gonflable; dans le deuxième type, on supporte le membre dans une position donnée au moyen d'une carcasse rigide; dans le troisième type, on supporte le membre par une structure externe rigide articulée en plusieurs points par des axes de façon à permettre une certaine mobilité des différtentes parties du membre l'une par rapport à l'autre, mais le sujet ne peut commander les mouvements de son membre que par une action externe, soit avec un moteur externe, soit par une action de son autre membre. Dans tous les cas, le membre est entièrement supporté et le jeu des articulations, quand il existe, est beaucoup plus limité que le jeu articulaire physiologique.

Au contraire, selon l'invention, le membre peut avoir tous les mouvements physiologiques normaux, les articulations pouvant jouer dans tous les sens physiologiquement admissibles et la commande du membre est effectuée par le sujet lui-même, sans l'aide de moteur externe.

Dans la demande de brevet FR-A-143 132, on a décrit un appareillage externe pour handicapés moteurs d'au moins un membre supérieur, ledit appareillage permettant au sujet de mouvoir son membre handicapé, caractérisé par le fait qu'il comporte, pour chaque membre handicapé:

a) d'une part, une structure thoraco-scapulaire constituée de deux sous-ensembles:

— en premier lieu, une épaulière formée d'au moins trois coquilles semi-rigides reliées entre elles de façon souple, la première étant disposée sur l'angle supérieur du pectoral et passant sur l'arête supérieure du trapèze, la seconde étant disposée sur la zone claviculo-trapézoïdale à la pointe de l'épaule, la troisième étant disposée au droit du scapulum, au moins un tendeur élastique étant prévu entre la seconde coquille et chacune des deux autres;

— en second lieu, un sous-ensemble d'amarrage disposé autour du torse et relié à l'épaulière précitée en au moins trois points d'accrochage répartis de part et d'autre de l'épaule;

b) d'autre part, un para-squelette du membre supérieur et de la main comprenant:

— en premier lieu, cinq éléments de liaison avec le membre disposés respectivement au niveau des parties humérales supérieure et inférieure, des parties radio-cubitales supérieure et inférieure et de la main, l'élément de liaison huméral supérieur comprenant la seconde coquille de l'épaulière et un lieu périphérique qui entoure le membre, chacun des quatre autres éléments de liaison comportant une coquille semi-rigide, réalisée en une ou plusieurs pièces, et au moins un lien périphérique qui entoure le membre;

— en second lieu, des moyens élastiques disposés entre deux des éléments de liaison sus-mentionnés comprenant:

  . un premier levier-ressort fonctionnant en flexion et en torsion, fixé entre la seconde coquille de l'épaulière et la coquille de l'élément de liaison huméral inférieur, sensiblement parallèlement à l'humérus le long de la bordure externe du membre,

  . un second levier-ressort analogue au premier, fixé entre la coquille de l'élément de liaison radio-cubital supérieur et la coquille de la main, sensiblement parallèlement au cubitus le long de la bordure interne du membre,

  . deux tendeurs élastiques disposés sur les bordures externe et interne du bras, entre les éléments de liaison huméral inférieur et radio-cubital supérieur, deux tendeurs élastiques disposés entre l'élément de liaison radio-cubital inférieur et l'élément de liaison de la main.

Il a été décrit dans le document DE-C-299 882 un élément de liaison de la main présentant des caractéristiques sensiblement similaires mais destiné à permettre, à lui seul, les mouvements de la main ou des doigts immobilisés suite à une blessure.

L'appareillage selon la présente invention est destiné, au contraire, à faire partie d'une orthèse complète du membre supérieur et n'est pas capable de jouer son rôle séparément.

Dans un mode préféré de réalisation de cet appareillage la coquille de l'élément de liaison radio-cubital inférieur est constituée de deux demi-coquilles destinés à recouvrir respectivement la partie radiale et la partie cubitale du membre lorsque l'appareil est en place, ces deux demi-coquilles étant séparées par un sillon au droit du cubitus lorsque l'appareil est en place, le mebre étant introduit dans la coquille par le côté opposé au sillon, les deux demi-coquilles étant serrées autour du membre par deux liens périphériques disposés aux deux extrémités supérieure et inférieure de ladite coquille; l'élément de liaison radio-cubitale intérieur ou supérieur recouvre environ le tiers de l'avant-bras lorsque l'appareil est en place.

Selon l'invention, l'élément de liaison de la main associé à un élément de liaison radio-cubital inférieur du type ci-dessus défini comporte trois segments, le premier segment destiné à recouvrir l'éminence thénar lorsque l'appareil est en place, le deuxième segment destiné à recouvrir les deuxième, troisième et quatrième carpiens et métacarpiens jusqu'à la base des doigts lorsque l'appareil est en place, et est caractérisé pasr le fait qu'il comporte un troisième segment destiné à recouvrir l'éminence hypothénar lorsque l'appareil est en place et, d'une part, un premier lien périphérique, disposé sur les premier et deuxième segments précités et destiné à entourer la main à l'exception du pouce au niveau de la partie inférieure des métacarpiens lorsque l'appareil est en place et, d'autre part, un deuxième lien périphérique destiné à entourer le carpe et passant sur les trois segments au voisinage du poignet lorsque l'appareil est en place, ledit deuxième lien périphérique étant relié à l'élément de liaison radio-cubital inférieur par une bande de caoutchouc; l'élément de liaison de la main est relié à l'élément de liaison radio-cubital inférieur par deux tendeurs, l'un, entre la base du pouce et la zone radiale inférieure lorsque l'appareil est en place et, l'autre, entre la zone cubitale inférieure et la zone palmaire médiane du métacarpe, passant en diagonale au-dessus de la main lorsque l'appareil est en place; le second levier-ressort de l'appareillage est disposé en regard de l'espace entre le cubitus et le bord interne de l'avant-bras et il a son extrémité inférieure fixée sur le troisième segment de la coquille de l'élément de liaison de la main, un peu en avant du pisiforme lorsque l'appareil est en place.

Dans une première variante de réalisation de l'élément de liaison de la main, un lien porté par le premier segment de la coquille de la main entoure la base du pouce, les autres doigts de la main n'étant pas appareillés. Dans une deuxième variante , chaque doigt de la main est appareillé par un tendeur élastique croisé disposé entre une borne d'appui placé au-dessus du métacarpe et une borne d'appui placée au-dessus de la seconde phalange; la borne d'appui placée au niveau du métacarpe est portée par le segment de la coquille de la main, qui lui correspond, et la borne d'appui placée au niveau de la seconde phalange est portée par un doigtier, qui entoure l'extrémité du doigt; chaque borne d'appui comporte, de préférence, un élément triangulaire à orientation réglable susceptible de tourner autour d'un axe sensiblement perpendiculaire à l'os qu'il surmonte.

Pour mieux faire comprendre l'objet de l'invention, on va décrire maintenant, à titre d'exemple purement illustratif et non limitatif, un mode de réalisation d'appareillage selon la demande de brevet français 82-18902 équipé d'un élément de liaison de la main selon l'invention er représenté sur le dessin annexé.

Sur ce dessin:

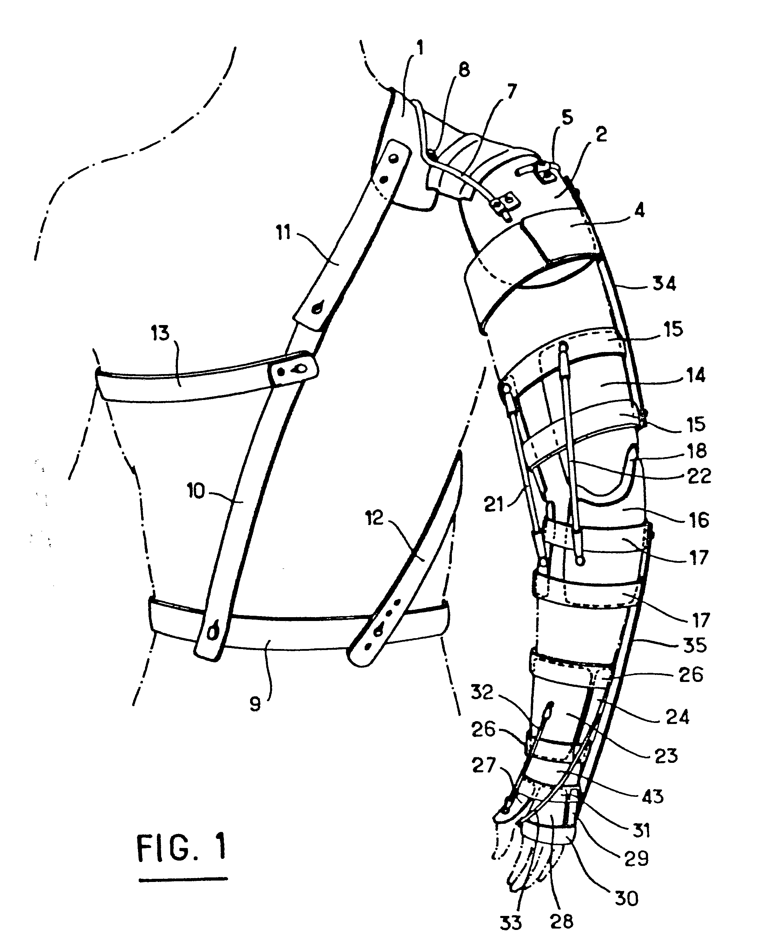

la figure 1 représente une vue schématique antérieure d'un appareillage selon l'invention porté par un sujet handicapé moteur du bras gauche;

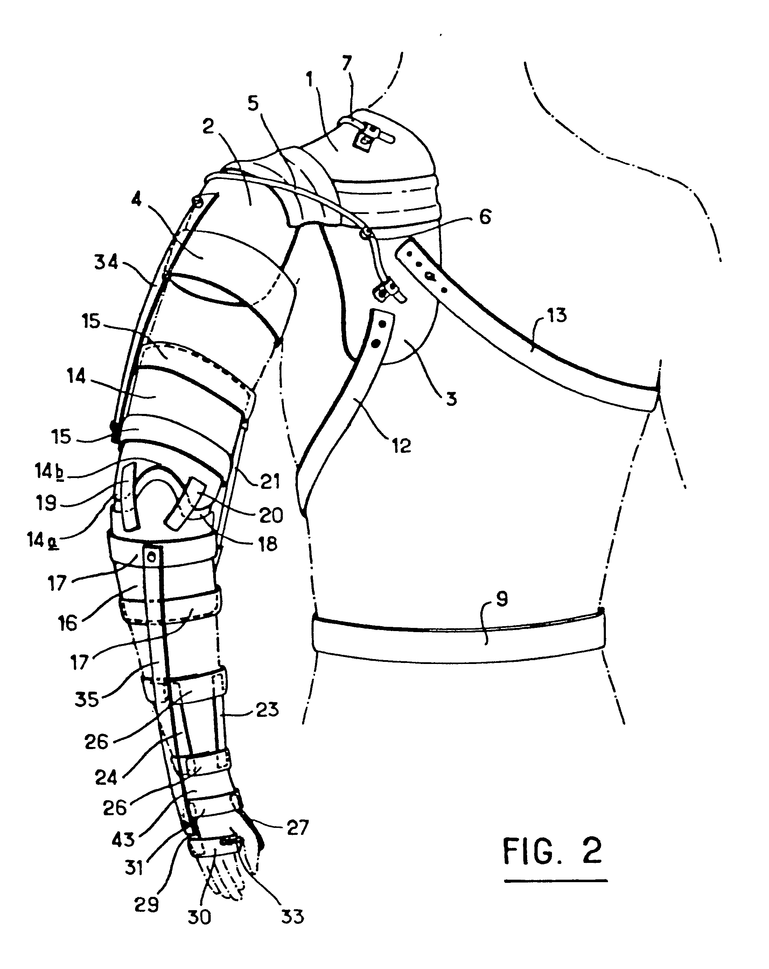

la figure 2 représente une vue schématique postérieure de l'appareillage de la figure 1;

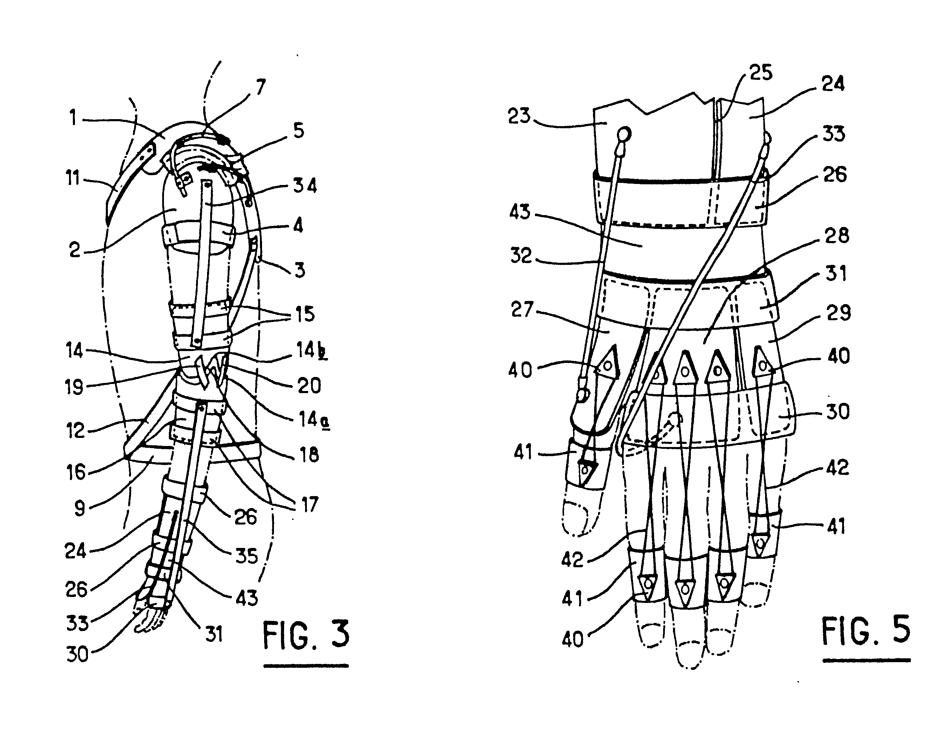

la figure 3 représente une vue latérale externe de l'appareillage des figures 1 et 2;

la figure 4 représente, en perspective, une première variante de la coquille de la main, dans laquelle le premier segment est équipé d'un lien pour supporter le pouce;

la figure 5 représente, vue en plan, une deuxième variante de réalisation de l'élément de liaison de la main, dans laquelle chaque doigt est appareillé avec un tendeur croisé;

la figure 6 représente schématiquement, en perspective éclatée, la réalisation d'une borne d'appui des tendeurs croisés de la figure 5.

En se référant au dessin, on voit que l'appareillage représenté comporte une structure thoraco-scapulaire constituée d'une épaulière et d'un sous-ensemble d'amarrage.

L'épaulière est formée de trois coquilles semirigides en matière plastique moulée. La première coquille 1 est disposée sur l'angle supérieur du pectoral et passe sur l'arête supérieure du trapèze. La seconde coquille 2 est disposée sur la zone claviculo-trapézoïdale à la pointe de l'épaule. La troisième coquille 3 est disposée au droit du scapulum, c'est-à-dire sur l'omoplate. Ces trois coquilles 1, 2, 3 sont reliées entre elles par des bandes de caoutchouc, mais elles sont séparées l'une de l'autre par des espaces suffisants pour permettre leur mouvement relatif. La forme de ces coquilles est adaptée anatomiquement au sujet qui doit les porter.

La coquille 2 de l'épaulière est solidaire d'un lien périphérique 4, qui est destiné à entourer élastiquement la partie supérieure du bras, le serrage étant assuré par une fermeture adhésive du type connu sous la dénomination commerciale «VELCRO», par exemple. La coquille 2 est, en outre, reliée à la coquille 3 par un tendeur 5, qui s'accroche sur la coquille 2 en un point de la zone postéro-supérieure de cette coquille, et sur la coquille 3 au droit de la partie sous-épineuse de l'omoplate, sensiblement dans la région centrale de l'omoplate. Le tendeur 5 passe sur une poulie 6, portée par la coquille 3 en un point situé au niveau de l'équipe de l'omoplate, sensiblement au tiers à partir de l'extérieur.

La coquille 2 est également reliée à la coquille 1 par un tendeur 7, qui est accroché sur la coquille 2 en un point de la partie supérieure de cette coquille qui est au niveau de la gouttière du biceps. Le tendeur 7 est accroché sur la coquille 1 en un point situé au droit de la partie sus-épineuse interne de l'omoplate, dans la zone d'insertion du trapèze. Le tendeur 7 passe sur une poulie 8, portée par la coquille 1 et disposée au droit de la coracoïde.

La bande de caoutchouc, qui relie les coquilles 1 et 2, est disposée vers l'avant, du deltoïde moyen vers la clavicule. La bande de caoutchouc qui assure la liaison entre la coquille 2 et la coquille 3 est disposée vers l'arrière, du deltoïde moyen vers l'épine de l'omoplate. Les formes des coquilles 1, 2, 3 et les liaisons établies ménagent un angle de déplacement du bras par rapport à l'épaule d'environ 45° vers l'avant ou vers l'arrière. La bande de caoutchouc, qui assure la liaison entre les coquilles 1 et 3, est disposée sensiblement au-dessus de l'épine de l'omoplate.

Le deuxième sous-ensemble de la structure thoraco-scapulaire est le sous-ensemble d'amarrage. Ce sous-ensemble comporte une ceinture 9 entourant le sujet au niveau de la taille. A cette ceinture est relié une liaison antérieure constituée d'un levier-ressort thoracique 19 et d'un raccord élastique 11. Le levier-ressort thoracique 10 s'accroche sur la ceinture 9 au niveau de l'épine iliaque du côté opposé au membre appareillé; il est lié au raccord élastique 11 au niveau de l'apendice xiphoïde, c'est-à-dire de la pointe du sternum. Le raccord élastique 11 est accroché sur la coquille 1 de l'épaulière en un point situé sur la partie antéro-inférieure de la coquille 1, au-dessous de la clavicule. Le raccord élastique 11 est constitué d'une bande élastique; le levier-ressort thoracique 10 est constitué d'une lame métallique en acier de type «XC75», la lame ayant une épaisseur de 0,5 mm et une largeur de 25 mm; la longueur de la lame est fonction de l'anatomie du sujet. Il serait possible d'envisager de remplacer ce levier-ressort 10 par une transmission thoracique, qui serait uniquement constituée d'une courroie, mais le résultat semble moins bon dans ce cas quant à la transmission de l'énergie du moteur scapulaire sur le membre handicapé.

La ceinture 9 est raccordée par une première liaison élastique latérale 12 avec la coquille 3 de l'épaulière. La liaison élastique latérale est constituée d'une bande élastique; elle s'accroche sur la ceinture 9 au niveau de l'épine iliaque du côté du membre appareillé; elle s'accroche sur la coquille 3 au niveau de la pointe de l'omoplate.

Entre, d'une part, la zone de jonction du levier-ressort 10 avec le raccord élastique 11 et, d'autre part, la coquille 3, se trouve une deuxième liaison latérale 13, constituée d'une bande élastique. Le point d'attache de la bande 13 sur la coquille 3 est disposé dans la zone supérieure interne de la coquille 3, légèrement au-dessous de l'extrémité interne de l'épine de l'omoplate. Le positionnement de la bande 13 est tel que le mamelon du côté opposé au membre appareillé reste dégagé.

L'appareillage selon l'invention associe la structure thoraco-scapulaire ci-dessus décrite à un parasquelette du membre supérieur et de la main. Ce parasquelette comporte cinq éléments de liaison avec le membre handicapé. L'élément de liaison huméral supérieur est constitué de la coquille 2 et du lien périphérique 4; il s'étend sensiblement sur un tiers de la longueur du bras. L'élément de liaison huméral inférieur est constitué d'une coquille 14, qui présente du côté interne du bras une ouverture permettant l'introduction du membre. La coquille 14 est maintenue sur le membre au moyen d'un lien périphérique 15 et l'ensemble 14, 15 recouvre la partie humérale inférieure du bras, sur environ un tiers de la longueur du bras. Le lien périphérique 15 est constitué de deux bandes analogues à la bande 4, disposées aux extrémités supérieure et inférieure de la coquille 14.

L'élément de liaison de la partie radio-cubitale supérieure comporte, également, une coquille 16 analogue à la coquille 14. La coquille 16 est également ouverte du côté de la partie intérieure de l'avant-bras; elle est fixée sur l'avant-bras par un lien périphérique 17, constitué de deux bandes élastiques analogues à la bande 4, les deux bandes étant placées aux extrémités supérieure et inférieure de la coquille 16. Les coquilles 14 et 16 sont séparées au niveau du coude par un espace 18, qui a une largeur sensiblement constante et égale à 8 mm. Pour permettre l'articulation de l'avant-bras par rapport au bras, la bordure inférieure de la coquille 14 présente deux formes convexes arrondies 14a au droit de l'épicondyle et de l'épitrochlée, ces deux formes convexes étant séparées par une concavité arrondie 14b au droit de l'olécrane. La bordure supérieure de la coquille 16 présente une forme complémentaire pour que l'espace articulaire 18 reste d'une largeur sensiblement constante. Cette découpe permet un mouvement relatif de la coquille 16 par rapport à la coquille 14, qui correspond à l'articulation du coude. Les coquilles 14 et 16 sont liées par deux tendeurs 19 et 20, disposés de part et d'autre de l'olécrane, au-dessus de l'espace articulaire 18. En outre, les coquilles 14 et 16 sont reliées entre elles par deux tendeurs élastiques 21 et 22, disposés respectivement dans le creux du coude, sur les bordures interne et externe du membre.

L'élément de liaison radio-cubital inférieur comporte une coquille constituée de deux demi-coquilles 23-24 recouvrant respectivement la partie radiale et la partie cubitale de l'avant-bras. Ces deux demi-coquilles sont séparées par un sillon 25 et elles sont serrées autour du membre par deux liens périphériques 26, disposés aux deux extrémités supérieure et inférieure de ladite coquille 23, 24. L'élément de liaison radio-cubital inférieur s'étend sur sensiblement le tiers inférieur de l'avant-bras. Le membre est introduit entre les deux demi-coquilles 23, 24 par le côté opposé au sillon 25.

L'élément de liaison de la main comporte une coquille constituée de trois segments 27, 28, 29. Le segment 27 recouvre l'éminence thenar. Le segment 28 recouvre les deuxième, troisième et quatrième carpiens et métacarpiens jusqu'à la base des doigts. Le troisième segment 29 recouvre l'éminence hypothenar. Ces trois segments sont maintenus sur le calice de la main au moyen de deux liens périphériques. Un premier lien périphérique 30 est disposé sur les segments 28 et 29, au niveau de la partie inférieure des métacarpiens, et il entoure la main à l'exception du pouce. Un deuxième lien périphérique 31 entoure le carpe et passe sur les trois segments 27, 28, 29, au voisinage du poignet. Les liens périphériques 30 et 31 ont une largeur d'environ 2 cm. Le premier lien périphérique 30 est relié élastiquement à la zone cubitale inférieure de la demi-coquille 24 par un tendeur élastique 33 qui passe en diagonale au-dessus du carpe et du métacarpe et se fixe dans la partie palmaire du lien 30 sensiblement au droit de la base du majeur. Un tendeur élastique 32 est disposé entre la zone radiale inférieure de la demi-coquille 23 et le segment 27, au niveau de la base du pouce. L'élément de liaison de la main est relié à l'élément de liaison radio-cubital inférieur par une bande de caoutchouc disposée au-dessus du poignet.

Toutes les coquilles 14, 16, 23, 24, 27, 28, 29 du para-squelette sont réalisées en matière plastique moulée en fonction de l'anatomie du sujet. Tous les liens périphériques 15, 17, 26, 30, 31 sont analogues au lien 4.

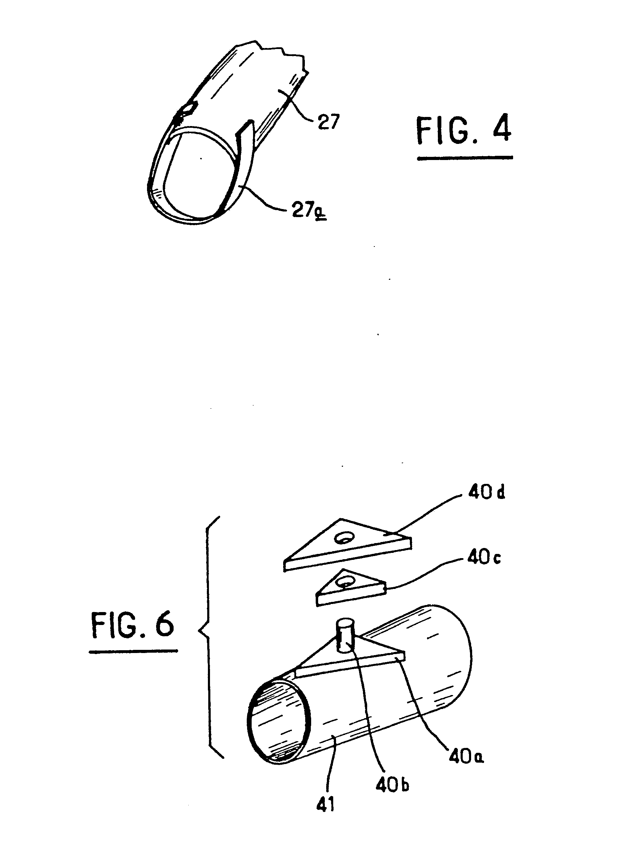

Dans la première variante représentée sura la figure 4, le segment 27 est équipé d'un lien 27a formant une anse et entourant la base de la colonne du pouce. Ce lien 27a peut être constitué d'une bande élastique insérée à l'extrémité inférieure du segment 27. Les autres doigts ne sont pas appareillés.

Dans une seconde variante, représentée sur les figures 5 et 6, chacun des doigts de la main est appareillé. Dans ce cas, chacun des segments 27, 28, 29 porte, au droit de chacun des métacarpiens, une borne d'appui désignée par 40 dans son ensemble. Le segment 27 porte donc une borne 40; le segment 28 porte trois bornes 40; et le segment 29 porte une borne 40. Toutes les bornes 40 sont identiques. Chaque borne 40 est constituée d'une embase 40a de forme triangulaire supportant un axe cylindrique 40b sensiblement perpendiculaire à l'os qu'il surmonte. Sur l'embase 40a repose une came triangulaire 40c, qui est enfilée sur l'axe 40b et qui est maintenue en place contre l'embase 40a par un couvercle triangulaire 40d de même forme et de même dimension que l'embase 40a, le couvercle 40d étant également enfilé sur l'axe 40b. L'empilement 40a, 40c, 40d est maintenu serré, par exemple par encliquetage du couvercle 40d sur le sommet de l'axe 40b. La position de la came 40c entre l'embase 40a et le couvercle 40d est réglable en rotation autour de l'axe 40b, mais en raison du serrage de l'empilement, cette position est maintenue après réglage par friction de la came 40c entre l'embase 40a et le couvercle 40d. On peut prévoir que les surfaces en regard soient granitées ou striées pour assurer une bonne friction.

Chacun des doigts de la main est équipé au niveau de la deuxième phalange d'un doigtier 41 ayant une forme légèrement tronconique. Chaque doigtier 41 comporte une borne d'appui 40 identique à celle qui à été précédemment décrite. Toutes les bornes 40 sont disposées sur le dessus de la main. Pour chacun des doigts de la main, un tendeur élastique 42 est mis en place entre la borne d'appui 40 située au niveau de la deuxième phalange et la borne d'appui 40 située au niveau de métacarpe. Le tendeur 42 est croisé en X et passe autour de la came 40c de chacune des bornes 40 qui lui sont associées. De la sorte, en orientant convenablement la came 40c de chacune des deux bornes d'appui, on peut régler la direction de l'effort de compression appliqué sur les articulations du doigt. Les tendeurs 42 constituent des freins élastiques à la fermeture de la main.

Le para-squelette qui vient d'être décrit comporte, en outre, de façon essentielle pour le fonctionnement de l'appareillage selon l'invention, deux leviers-ressorts constitués de lames métalliques analogues aux leviers-ressorts 10. Le premier levier-ressort 34 est fixé entre un point de la zone centrale de la coquille 2 de l'épaulière et la coquille 14; il est sensiblement parallèle à l'humérus, le long de la bordure externe du bras. Le second levier-ressort 35 est fixé entre la coquille 16 et le segment 29; il est sensiblement parallèle au cubitus, en regard de l'espace compris entre le cubitus et le bord interne de l'avant-bras; son accrochage sur le segment 29 s'effectue un peu en avant du pisiforme.

On constate que l'appareillage ci-dessus décrit permet à un handicapé moteur des membres supérieurs de retrouver une certaine mobilité de son membre handicapé.

En effet, les tensions des différents tendeurs des leviers-ressorts de l'appareillage sont réglées de façon qu'en position de repos l'avant-bras fasse environ un angle de 30° avec le bras et que le poignet soit en légère flexion dorsale (environ 10° vers le haut); en outre, les doigts sont en légère flexion palmaire (environ 15° vers le bas) et le pouce est en abduction de façon à ouvrir la pince de préhension.

Lorsque le sujet met son épaule en arrière, il ouvre le cône scapulaire constitué par l'omoplate et la clavicule, ce qui provoque la montée de l'humérus. Dans ce mouvement, le bras de levier de l'action du poids de l'avant-bras par rapport à l'articulation du coude augmente et il en résulte une légère extension par ouverture de l'angle du coude, les tendeurs 21, 22 freinant cette ouverture. Sous l'effet du poids, la main a tendance à descendre vers le bas, ce qui est freiné par les tendeurs 32, 33. La main a tendance à se fermer, ce qui est freiné par les tendeurs 42.

Si le sujet monte son épaule vers le haut et vers l'avant, on réalise un mouvement de retour par rapport au mouvement précédent. L'humérus est tiré en arrière; le bras de levier du poids de l'avant-bras par rapport au coude diminue et sous l'action des tendeurs 21, 22 l'avant-bras remonte, ce qui ferme légèrement l'angle du coude. La main tourne légèrement en tendant à venir paume en l'air et les doigts s'ouvrent.

On constate donc que le moteur scapulaire permet, en combinant les différents mouvements possibles de l'épaule, la commande de l'appareillage selon l'invention. Les mouvements de l'humérus, quand le sujet déplace son épaule, sont non seulement commandés par les attaches physiologiques, mais également par le levier-ressort 34 qui, étant fixé sur la coquille 2 de l'épaulière, subit les efforts de traction appliqués sur l'épaulière par les moyens d'accrochage 9, 10, 11, 12, 13. Le mouvement est transmis du bras à la main par le levier-ressort 35. On neutralise ainsi d'une façon dynamique le poids du membre handicapé.

On a constaté que le travail du membre ainsi appareillé permet la réactivation des unités motrices, ce qui accélère la récupération du geste de préhension. En outre, le sujet qui utilise un tel appareillage et mobilise son membre handicapé recouvre d'une façon spectaculaire une grande partie de la sensibilité du membre.

Il est bien entendu que le mode de réalisation ci-dessus décrit n'est aucunement limitatif et pourra donner lieu à toutes modifications désirables, sans sortir pour cela du cadre de l'invention tel que défini par les revendications.

La présente demande décrit des objets qui sont revendiqués dans EP-A-143 132.

## Revendications

1. Appareillage externe pour handicapé moteur de la main comportant d'une part, un élément de liaison radio-cubital inférieur destiné à recouvrir l'avant bras lorsque l'appareil est en place, d'autre part un élément de liaison de la main qui comporte un premier segment (27) destiné à recouvrir l'éminence thénar lorsque l'appareil est en place et un deuxième segment (28) destiné à recouvrir les deuxième, troisième et quatrième carpiens et métacarpiens jusqu'à la base des doigts lorsque l'appareil est en place, caractérisé par le fait que l'élément de liaison radio-cubital est constitué de deux demi-coquilles (23, 24) destiné à recouvrir respectivement la partie radiale et la partie cubitale de l'avant-bras lorsque l'appareil est en place, ces deux demi-coquilles étant séparées par un sillon (25) au droit du cubitus lorsque l'appareil est en place, l'avant-bras étant introduit dans la coquille constituée des deux demi-coquilles (23, 24) par le côté opposé au sillon (25), les deux demi-coquilles (23, 24) étant serrées autour du membre par deux liens périphériques (26) disposés aux extrémités supérieures et inférieures de la coquille; et que l'élément de liaison de la main comporte un troisième segment (29) destiné à recouvrir l'éminence hypothénar lorsque l'appareil est en place, et, d'une part, un premier lien périphérique (30) disposé sur le premier et deuxième segments (27, 28) et destiné à entourer la main à l'exception du pouce au niveau de la partie inférieure des métacarpiens lorsque l'appareil est en place, et, d'autre part, un deuxième lien périphérique (31) destiné à entourer le carpe et passant sur les trois segments (27, 28, 29) au voisinage du poignet lorsque l'appareil est en place, le deuxième lien périphérique (31) étant relié à l'élément de liaison radio-cubital inférieur (23, 24, 26) par une bande de caoutchouc (43).

2. Appareillage selon la revendication 1, caractérisé par le fait que l'élément de liaison radio-cubital inférieur (23, 24, 26) recouvre environ le tiers de l'avant-bras lorsque l'appareil est en place.

3. Appareillage selon l'une des revendications 1 ou 2, caractérisé par le fait que l'élément de liaison de la main (27, 28, 29, 30, 31) est relié à l'élément de liaison radio-cubital inférieur (23, 24, 26) par deux tendeurs (32, 33) disposés l'un (32) entre la base du pouce et la zone radiale inférieure, l'autre (33), entre la zone cubitale inférieure et la zone palmaire médiane du métacarpe, passant en diagonale au-dessus de la main lorsque l'appareil est en place.

4. Appareillage selon l'une des revendications 1 à 3, caractérisé par le fait que le premier segment (27) de la coquille de l'élément de liaison de la main porte un lien (27a), qui entoure la base du pouce lorsque l'appareil est en place.

5. Appareillage selon l'une des revendications 1 à 4, caractérisé par le fait que chaque doigt de la main peut être appareillé par un tendeur élastique croisé (42), disposé entre une borne d'appui (40) placée au-dessus du métacarpe et une borne d'appui (40) placée au-dessus de la seconde phalange lorsque l'appareil est en place.

6. Appareillage selon la revendication 5, caractérisé par le fait que la borne d'appui (40) placée au niveau du métacarpe lorsque l'appareil est en place est portée par le segment de la coquille de la main, qui lui correspond, et que la borne d'appui (40) placée au niveau de la seconde phalange lorsque l'appareil est en place est portée par un doigtier (41), qui entoure l'extrémité du doigt.

7. Appareillage selon l'une des revendications 5 ou 6, caractérisé par le fait que chaque borne d'appui (40) comporte un élément triangulaire (40c) à orientation réglable susceptible de tourner autour d'un axe (40b) sensiblement perpendiculaire à l'os qu'il surmonte lorsque l'appareil est en place.

**Patentansprüche**

1. Externe Vorrichtung für in der Beweglichkeit ihrer Hand behinderte Personen, die umfasst:

zum einen ein unteres radio-kubitales Verbindungselement das dazu bestimmt ist, den Vorderarm zu umschliessen, wenn die Vorrichtung angelegt ist, und zum anderen ein Element zum Zusammenhalten der Hand, umfassend ein erstes Segment (27), welches dazu bestimmt ist, den Daumenballen zu umschliessen, wenn die Vorrichtung angelegt ist, ein zweites Segment (28), welches dazu bestimmt ist, die zweite, dritte und vierte Handwurzel und die Mittelhand bis zum Fingeransatz zu umschliessen, wenn die Vorrichtung angelegt ist, dadurch gekennzeichnet, dass das radio-kubitale Verbindungselement aus zwei Halbschalen (23, 24) besteht, welche dazu bestimmt sind, jeweils den radialen Teil und den kubitalen Teil des Vorderarmes zu umschliessen, wenn die Vorrichtung angelegt ist, wobei die beiden Halbschalen durch eine Rille (25) rechts der Elle geteilt sind, wenn die Vorrichtung angelegt ist, und wobei der Vorderarm an der der Rille (25) gegenüberliegenden Seite in das aus den beiden Halbschalen (23, 24) bestehende Gehäuse eingeführt wird und die beiden Halbschalen (23, 24) mit zwei Umfangsbändern (26), welche am oberen und unteren Ende der Halbschalen angeordnet sind, zugezogen werden; und dass das Element zum Zusammenhalten der Hand ein drittes Segment (29), das dazu bestimmt ist, den Daumenballen zu umschliessen, wenn die Vorrichtung angelegt ist und einerseits ein erstes Umfangsband (30), das an dem ersten und zweiten Segment (27, 28) angebracht ist und dazu bestimmt ist, die Hand mit Ausnahme des Daumens in Höhe des unteren Teils der Mittelhand zu umgeben, wenn die Vorrichtung angelegt ist, und andererseits ein zweites Umfangsband (31) umfasst, das dazu bestimmt ist, die Oberhand zu umschliessen und das über die drei Segmente (27, 28, 29) bis in die Nähe des Daumens verläuft, wenn die Vorrichtung angelegt ist, wobei das zweite Umfangsband (31) mit dem unteren radio-kubitalen Verbindungselement (23, 24, 26) durch ein Kautschukband (43) verbunden ist.

2. Vorrichtung nach Anspruch 1, dadurch gekennzeichnet, dass das untere radio-kubitale Verbindungselement (23, 24, 26) ungefähr ein Drittel des Vorderarmes umschliesst, wenn die Vorrichtung angelegt ist.

3. Vorrichtung nach Anspruch 1 oder 2, dadurch gekennzeichnet, dass das Element zum Zusammenhalten der Hand (27, 28, 29, 30, 31) mit dem unteren radio-kubitalen Verbindungselement (23, 24, 26) durch zwei Spannglieder (32, 33) verbunden ist, wobei sich das eine Spannglied (32) zwischen dem Daumenansatz und dem unteren Radialbereich und das andere Spannglied (33) zwischen dem unteren Kubitalbereich und dem mittleren Palmarbereich der Mittelhand befindet, und welche diagonal auf der Hand verlaufen, wenn die Vorrichtung angelegt ist.

4. Vorrichtung nach einem der Ansprüche 1 bis 3, dadurch gekennzeichnet, dass das erste Segment (27) des Gehäuses des Elements zum Zusammenhalten der Hand ein Band (27a) aufweist, das den Daumenansatz umschliesst, wenn die Vorrichtung angelegt ist.

5. Vorrichtung nach einem der Ansprüche 1 bis 4, dadurch gekennzeichnet, dass für jeden Finger der Hand ein elastisches gekreuztes Spannglied (42) vorgesehen sein kann, das sich zwischen einem oberhalb der Mittelhand angebrachtes Befestigungsstück (40) und einem oberhalb des zweiten Fingergliedes angebrachtes Befestigungsstück (40) befindet, wenn die Vorrichtung angelegt ist.

6. Vorrichtung nach Anspruch 5, dadurch gekennzeichnet, dass das Befestigungsstück (40), das sich in Höhe der Mittelhand befindet, wenn die Vorrichtung angelegt ist, durch das Segment des Gehäuses der Hand gehalten wird, welches ihm zugeordnet ist, und dass das Befestigungsstück (40), welches sich in Höhe des zweiten Fingergliedes befindet, wenn die Vorrichtung angelegt ist, durch einen Fingerling (41) gehalten wird, der das Ende des Fingers umschliesst.

7. Vorrichtung nach einem der Ansprüche 5 oder 6, dadurch gekennzeichnet, dass jedes Befestigungsstück (40) ein dreieckiges Element (40c) mit einstellbarer Orientierung aufweist, das sich um eine Achse (40b) drehen kann, welche im wesentlichen senkrecht zum Knochen ist, über dem sie liegt, wenn die Vorrichtung angelegt ist.

## Claims

1. An external appliance for handicapped motor of the hand, comprising on the one hand a lower radiocubital connection element intended to cover the forearm when the equipment is in place, on the other hand, an element for connection to the hand which comprises a first segment (27) intended to cover the thenar eminence when the equipment is in place, and a second segment (28) intended to cover the second, third and fourth carpals and metacarpals down to the base of the fingers when the equipment is in place, characterized by the fact that the radiocubital connection element is constituted by two half shells (23, 24) intended to cover respectively the radial part and the cubital part of the forearm when the equipment is in place, these two half shells being separated by a slot (25) to the right of the cubitus when the equipment is in place, the forearm being introduced into the shell constituted by the two half shells (23, 24) by the end opposite to the slot (25), the two half shells (23, 24) being locked about the member by two peripheral straps (26) disposed at the upper and lower ends of the shell; and in that the element for connecting to the hand comprises a third segment (29) intended to cover the hypothenar eminence when the equipment is in place, and, on the one hand, a first peripheral strap (30) disposed on the first and second segments (27, 28) and intended to surround the hand with the exception of the thumb in the vicinity of the lower part of the metacarpals when the equipment is in place, and, on the other hand, a second peripheral strap (31) intended to surround the carp and passing over the three segments (27, 28, 29) in the vicinity of the wrist when the equipment is in place, the second peripheral strap (31) being connected to the lower radiocubital connection element (23, 24, 26) by a rubber band (43).

2. Appliance according to claim 1 characterized by the fact that the lower radiocubital connection element (23, 24, 26) covers substantially a third of the forearm when the equipment is in place.

3. Appliance according to one of claims 1 or 2, characterized by the fact that the element (27, 28, 29, 30, 31) for connection to the hand is connected to the lower radiocubital connection element (23, 24, 26) by two tensioners (32, 33) disposed with one (32) between the base of the thumb and the lower radial zone, the other (33) between the lower cubital zone and the median palmar of the metacarp passing diagonally over the hand when the equipment is in place.

4. Appliance according to one of claims 1 to 3, characterized by the fact that the first segment (27) of the shell of the connection element to the hand carries a strap (27a), which surrounds the base of the thumb when the equipment is in place.

5. Appliance according to one of claims 1 to 4, characterized by the fact that each finger of the hand may be equipped with a crossed elastic tensioner (42) disposed between a bearing post (40) placed above the metacarp and a bearing post (40) placed above the second phalanx when the equipment is in place.

6. Appliance according to claim 5, characterized by the fact that the bearing post (40) placed in the vicinity of the metacarp when the equipment is in place is carried by the segment of the hand shell which corresponds to it, and that the bearing post (40) placed in the vicinity of the second phalanx when the equipment is in place is carried by a finger stall (41) which surrounds the end of the finger.

7. Appliance according to one of claims 5 or 6, characterized by the fact that each bearing post (40) comprises a triangular element (40c) of adjustable orientation capable of turning about an axis (40b) substantially perpendicular to the bone which it surmounts when the equipment is in place.

## FIG. 1

FIG. 2

FIG. 5

FIG. 3

FIG. 4

FIG. 6